# EUROPEAN PATENT SPECIFICATION

(11) **EP 2 068 950 B1**
(45) Date of publication and mention of the grant of the patent: **20.06.2012**
(21) Application number: 07861358.5
(22) Date of filing: 21.09.2007
(51) Int. Cl.: A61L 24/02, A61L 33/02

(54) **DIAMMONIUM PHOSPHATE AND OTHER AMMONIUM SALTS AND THEIR USE IN PREVENTING CLOTTING**
DIAMMONIUMPHOSPHAT UND ANDERE AMMONIAKSALZE SOWIE IHRE VERWENDUNG ZUR GERINNUNGSVERHINDERUNG
PHOSPHATE DE DIAMMONIUM ET AUTRE SELS D'AMMONIUM, ET LEUR UTILISATION POUR PREVENIR LA COAGULATION

(30) Priority: 21.09.2006 US 846312 P
(43) Date of publication of application: 17.06.2009
(73) Proprietor: Kyphon SÀRL, 2000 Neuchâtel (CH)
(72) Inventor: LEE, Samuel, Mountain View CA 94040 (US); LOUIE, Stephanie, San Mateo CA 94402 (US); SCHWARDT, Jeffrey D., Palo Alto CA 94306 (US); SLATER, Thomas A., Mountain View CA 94043 (US); WENZ, Robert, 61206 Wollstadt (DE)
(74) Representative: O'Connell, Maura
(86) International application number: PCT/US2007/020506
(87) International publication number: WO 2008/039382

(56) References cited:
- EP-A- 1 120 123
- US-A- 4 273 873
- US-A- 4 708 951
- US-A1- 2005 142 211

## Description

### Field of the Invention

The present invention relates to products, methods, and processes of various ammonium salts and their use as anti-coagulants.

### Background of the Invention

The coagulation mechanism of blood involves a proteolytic cascade pathway wherein each enzyme in the pathway is present in blood plasma as a zymogen (an inactive protein) which undergoes proteolytic cleavage to generate its active counterpart. The coagulation pathway contains a series of factors that positively and negatively provide feedback loops which controls the coagulation activation process.

There are instances where it is desired that coagulation of blood be inhibited. Among these instances where an anti-coagulant may be used are when medical devices or medical compositions and/or pharmaceutical compositions are used that contact circulating blood. When employing medical devices, or alternatively, medical compositions or pharmaceutical compositions that contact circulating blood, there is a.n increased risk of blood clot formation, which may potentially lead to serious medical problems such as cardiac arrest, cerebrovascular accidents, pulmonary embolism, myocardial infarction, reduction in arterial blood pressure, and other adverse conditions.

Anti-coagulation (or anti-clotting) drugs exist. Heparin is a polysacharride molecule anti-coagulation drug that is either given systemically or used to coat blood-contacting implants to reduce the risk of blood clot formation. However, heparin tends to be rather expensive (relative to inorganic salts) and may not be appropriately used in all pharmaceutical compositions. Further, intramuscular injection of heparin is undesirable in most medical situations due to the possibility of hematoma formation. Heparin, generally, is degraded and rendered inoperative under acidic conditions (for example, it is degraded when exposed to the acidic conditions in the stomach). Heparin also is metabolized in the liver by heparinase to generate uroheparin, which demonstrates only slight anti-thrombin activity. Accordingly, the present invention addresses some of the drawbacks that are present with using other anti-coagulants generally and heparin in particular.

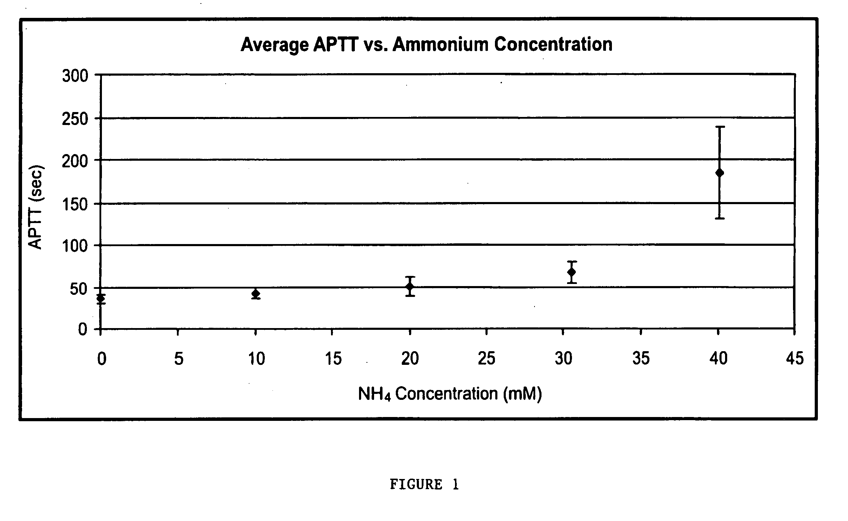

### Brief Description of the Several Views of the Drawing

The figure shows the average APTT (activated partial thromboplastin time) vs. ammonium concentration for an exemplary embodiment showing that as the concentration of ammonium salt increases the anticoagulant effects increase.

### Detailed Description of the Invention

The present invention relates to certain organic and inorganic ammonium salts that can be used as anti-coagulants. The invention provides an ammonium salt for use according to Claim 1. An ammonium salt is disclosed that can leach (e.g., diffuse, release, etc.) from a medical device while in contact with circulating blood that delays or prevents the formation of blood clots and lowers the risk of blood clots circulating to the brain, heart and lungs. In another variation, an ammonium salt is coated on the surface, or embedded within the body of a medical device for placement or implantation within a patient's body. In this variation, the ammonium salt may leach, diffuse, and/or release into the circulatory system of an individual and act as an anticoagulant. These ammonium salts in reducing and/or preventing blood clots may improve the safety of many medical procedures.

In an embodiment, any of a plurality of medical devices, implants, or compositions that are introduced into an individual can have ammonium salts incorporated into the medical device. For example, medical devices and implants such as cannulas, stents, catheters, grafts, heart valves, electrodes, balloons, patches, sutures and other medical devices and implants can have ammonium salts associated with them that allow the ammonium salt to leach, diffuse, and/or release into the individual. The ammonium salts and the associated medical device and implant can be designed so that slow release of the salts occur over time or a faster release of the ammonium salts may occur.

In another embodiment, the present invention relates to the release of a diammonium phosphate compound, composition, or solution containing a diammonium phosphate compound from bone cements during the setting phase of these bone substitutes. In an embodiment, when bone cement products containing ammonium salts are administered to patients, there is a concomitant delay or prevention of the coagulation process. In this regard, the release of a diammonium phosphate solution in this invention has been shown to delay and/or prevent clotting in whole sheep blood, whole human blood, and human plasma.

Thus, in an embodiment, the present invention relates to the use of ammonium phosphate salt, a diammonium phosphate salt and/or certain other ammonium salts and ammonium ions in medical devices, in implants and/or in compositions (e.g., bone cements) given to individuals such as patients in a medical context to prevent clots from forming.

In an alternative embodiment, the present invention relates to an anti-thrombogenic, clot prevention, blood compatible ammonium salt containing solution that can be administered to an individual that prevents or delays blood clotting. It is contemplated that this ammonium salt solution can be added either directly to blood or can be added indirectly to blood (for example, through passive diffusion or leaching) to aid in delaying or preventing blood clots from forming.

Several additional embodiments of the present invention are described below. These include:
1. The use of ammonium salts such as diammonium phosphate in bone substitutes. In a variation of this embodiment, aqueous diammonium phosphate solution is used as a phosphate ion source for an *in situ* setting reaction for a bone cement, such as a bone cement that contains both a powder and a liquid. The combination of a solution containing the ammonium salt with a powder component containing other components initiates a crystallization and precipitation reaction that results in a solid crystalline bone substitute. During the setting reaction, some of the excess ammonium salt, such as a diammonium phosphate salt, leaches out from the solid crystalline bone substitute mass to effectively create a localized area in the body that undergoes delayed blood clotting and/or alternatively, precludes blood clotting entirely.
2. The use of an ammonium salt in a non-resorbable polymeric extruded medical device: As an example of this embodiment, catheters can be formed by the extrusion of a polymer. An inorganic salt such as an ammonium salt can be included in a polymeric extrusion by introducing a concentrated solution containing the dissolved salt or a solid precipitate of the salt into the polymer melt mixture. A blood contacting catheter made in this manner can be configured such that when it contacts blood (an aqueous mixture), the incorporated ammonium salt, such as an ammonium phosphate or a diammonium phosphate salt dissolves and produces a local ammonium phosphate (or diammonium phosphate) solution effectively delaying and/or preventing potentially harmful blood clots.
3. The use of ammonium salts in a non-bioresorbable polymeric molded or thermoformed medical device: As an example of this embodiment, a polymeric molded or thermoformed component of a blood contacting medical device (for example, a blood filter, a drip chamber, a blood oxygenator, and the like) can be produced such that an ammonium salt, such as an ammonium phosphate salt or diammonium phosphate salt is available at the surface of the component. One means of doing this is by adding either a concentrated solution containing the dissolved salt or a solid precipitate of the salt on to the polymer or on to the mold during its molding or thermoforming process. Alternatively, the solid precipitate of the salt could be added directly to the polymer or polymer melt. In a further variation, the ammonium salt can be added to the polymeric mold after the polymer has been molded.
   Accordingly, in a variation of this embodiment, methods can be employed so that the solid precipitate of the salt occurs primarily at the surface of the medical device. Alternatively, the solid precipitate can be uniformly distributed throughout the medical device (such as by moving in the salt precipitate prior to the molding or thermoforming process). Whether the salt precipitate is primarily at the surface or uniformly distributed throughout the molded device, which blood contacts the surface of the component, the ammonium phosphate, or other ammonium salt dissolves and produces a local ammonium phosphate solution effectively delaying and/or preventing potentially harmful blood clots and/or preserving a clot free blood contacting surface.
4. Use in a bioresorbable polymeric implant: Some implantable medical devices are intended to resorb over time. An example of this embodiment is a bioresorbable suture constructed of PLA/PGA (polylactic acid/polyglycic acid) or other similar polymeric composition. Improved thrombogenicity of a suture could be obtained by incorporating ammonium phosphate, diammonium phosphate and/or another ammonium salt into the polymer by any of the methods described above.
5. The use of a controlled release of the ammonium phosphate, or other ammonium salt: As an example of this embodiment, the ammonium phosphate, diammonium phosphate and/or other ammonium salt is contained within a bioresorbable polymer, gel or hydrogel, or permeable membrane such that a controlled release of the salt occurs through dissolution into the aqueous blood. Such an implant could be permanently implanted, or temporarily implanted if the container is not bioresorbable, for the purpose of creating a localized area where blood clotting is delayed and/or prevented. This could be done to provide a non-thrombogenic environment or window to accomplish a medical procedure.
6. In another embodiment, one can use the dissolution behavior of Mg(NH₄)PO₄ as a solid, where Mg²⁺, PO₄³⁻, and more preferably NH₄⁺ ions are leached from the solid. The solid can be formed as a cementitious paste which hardens *in situ.* This is done by addition of an aqueous solution of (NH₄)₂HPO₄ to either Mg₃(PO₄)₂, or MgHPO₄ anhydrous or its various hydrates or mixtures thereof. Such a cementious paste forms Struvite as a solid and will slowly degrade in body fluids by dissolution. This alternate embodiment may provide mechanical support as a bone void filler with the ability to prevent blood clotting. Alternatively, the solid material can be milled down to various particle sizes achieving distinct surface geometries, which may allow the determination of the solubility profile, when it is admixed into for example, extruded polymers or resorbable polymers as a filler. This admixture may also have the ability to prevent blood clots. Such an admixture of slowly degrading Struvite (Mg(NH₄)PO₄) into polymeric medical devices, such as catheters, will prevent blood clotting over a broad time range since it has a slow degradation profile.

In an embodiment, the present invention relates to an inexpensive, simple salt that delays and/or prevents blood contact formation that can be incorporated into any medical product that contacts blood, or alternatively, that can be used in a wide variety of delivery vehicles. These devices may be any that contact blood *in vivo,* such as described above; any that contacts extracorporeal blood such as during transfusions, open-heart surgeries, etc.; that contact blood specimens *in vitro* or *ex vivo* such as blood in specimen tubes (which traditionally have often been pre-coated with heparin), syringes, blood donation storage bags and/or other blood laboratory disposables. Vehicles for delivering ammonium phosphate, diammonium phosphate and/or certain other ammonium salts can also be used in these scenarios.

The medical devices of the present invention are described with reference to the treatment of humans, however, it should be recognized that the above medical devices can also be used on animals, such as pets, livestock, other primates, and other animals. Accordingly, the above and below described embodiments such as products, methods and processes of use can be used in veterinary applications as well as their use in humans.

One embodiment of the present invention uses smaller doses of ammonium salts such as small doses of ammonium phosphate and diammonium phosphates. Generally, the human body rapidly metabolizes ammonia. Therefore when the amounts of ammonium salt used are relatively small, they can be metabolized and/or resorbed by the body without the possibility of any potential toxic side effects.

Possible ammonium salts that can be used in the present invention include diammonium phosphate salts, ammonium phosphate salts, ammonium chloride salts, ammonium bromide salts, ammonium iodide salts, ammonium sulfate salts, ammonium nitrate salts, ammonium thiocyanate salts, ammonium sulfaminate salts, and mixtures thereof.

Ammonium salts that do not fit within the definition of ammonium salts in the present invention include ammonium salts of citrate, ammonium salts of heparin, ammonium salts of tartrate, and ammonium salts of oxalate. However, it should be recognized that these ammonium salts can be used in connection with the above enumerated ammonium salts.

Moreover, other anticoagulants can be used in conjunction with the ammonium salts of the present invention, including but not limited to Anisindione, warfarin (Coumadin), heparin, Sintrom (Acenocoumarol), Warfilone, Miradon, 1,3-indanediones, antithrombin, protein C, and thrombomodulin.

In an embodiment, the ammonium salts of the present invention can be taken orally. Alternatively, the ammonium salts of the present invention can be administered when performing surgery or some other procedure wherein luminal regions and the interior of the body is exposed. Alternatively, the ammonium salts of the present invention can be administered percutaneously, by entrance through a sphincter or some other body passage, or alternatively by some other recognized passage wherein medicines can be introduced to the circulatory system.

In an embodiment, the ammonium salt of the present invention is present in a bone cement. In a variation of this embodiment, the bone cement is made from a liquid portion and a powder portion, wherein the bone cement is made by mixing the liquid portion and the powder portion together. The ammonium salt can be present in the liquid portion or in the powder portion prior to mixing the liquid portion and the powder portion together.

It is contemplated and therefore within the scope of the present invention that the bone cement may contain additional components. These additional components include one or more antibiotics such as gentamicin, gentamicin sulfate, erythromycin, tobramycin, vancomycin, cefazolin, oxacillin, cefotaxime, colistin, clindamycin, and/or fusidic acid. When the cement of the present invention foams, any antibiotic that is added to the mixture tends to be spread out uniformly throughout the cement. This leads to a more uniform release of the antibiotics when it is applied to bone. One reason why gentamicin sulfate is a suitable antibiotic for the present invention is because it is wide spectrum antibiotic that can be used to attack a large variety of bacteria.

In another variation, the ammonium salt can be added to a foaming cement so that the ammonium salt can leach out, release, or diffuse into a desired area of the body when the cement is inserted. The cement can be of a composition that allows the leaching, release, of diffusion of the ammonium salt at a desired time, for example, when the cement reaches a certain viscosity. Alternatively, the ammonium salt may be released when the cement reaches a different physical property, such as temperature, pH, or a certain concentration of a reaction product or some other property is attained. In general, the cement tends to be able to release ammonium salts when the cement is setting but when the cement is completely set, ammonium salt release tends to be somewhat limited.

Alternatively, and/or additionally, additional components that can be added to the bone cement of the present invention include one or more radiopacifier compounds such as barium sulfate, 2-[2',3',5'-triiodobenzoyl] ethyl methacrylate (TIBMA), 3,5-diiodine salicylic methacrylate (DISMA), and/or zirconium(IV) oxide. It is contemplated that other compounds that can be seen under fluoroscopic guidance can be used as radiopacifier compounds.

Additionally, anticancer agents can be added to the bone cement including but not limited to 6-mercaptopurine, methotrexate and/or cisplatin.

Other components that can be added include re-enforcing materials such as additional hydroxyapatite (HA) powder, K₂O-Na₂-CaO-MgO-SiO₂-P₂O₅ crystallized glass powder, other bioactive glasses, calcium phosphate, carbon, graphite, aramid, bone particles, bone chips, polyethylene, titanium, other metals, ultra high weight polyethylene, polymethylmethacrylate fibers in a cement matrix, tricalcium phosphate, and hydroxycarbonate apatite, and the like.

It is contemplated and therefore within the scope of the invention that the bone cement can be used in conjunction with one or more bone surgical screws, metal rods or plates (such as titanium rods or plates), NITINOL alloy structural devices, and/or other mechanical structural devices that add structural strength to the bone. When these structural devices are used, the cement may be used with or without one or more of the above-identified re-enforcing agents. When the bone cement formulation is used without one or more re-enforcing agents, the bone cement serves, in essence, as a lattice that allows accretion of bone into the macroscopic voids. The accretion of bone into the lattice (in combination with the bone cement formulation) may lead to bone that has greater structural strength.

### Examples

The effect of a material on blood clotting can be investigated *in vitro* by comparing the coagulation time of blood or plasma that does not contact a material containing an ammonium salt to the coagulation time of blood or plasma in contact with the material (containing the ammonium salt). Coagulation time of fresh whole blood can be determined with the Lee-White test by visually observing clot formation. Activation of the intrinsic coagulation pathway can be determined by measuring the activated partial thromboplastin time (APTT) as for example, shown in the figure. In this test, the time to formation of a fibrin clot in re-calcified citrated plasma is measured using a coagulation analyzer instrument.

Several studies have been performed to investigate the effects of a sample, which is a composition of bone cements that contain radiopaque calcium phosphate bone substitute and its liquid component, a diammonium hydrogen-phosphate solution (collectively referred to as Sample A), on clotting. A study suggested that setting bone cement samples that have diammonium salts in them increase the clotting time of whole sheep blood while set samples that have ammonium salts in them do not appear to affect the clotting time (see the below study 1). Since ammonium ions are the primary chemical component released from the material during setting, the effect of the Sample A on clotting time of whole sheep blood was investigated (see the below Study 2). Finally, the effects of different concentrations of the ammonium solution on clotting time of human plasma were investigated using the more precise APTT test (see the below Study 3).

### Study 1

The purpose of this study was to investigate the effect of sample A on the Lee-White clotting time of whole sheep blood.

A sample of setting bone substitute or a sample of set bone substitute or a glass ball (control article) was placed into a blood collection tube. Blood was drawn from the jugular vein of a donor sheep and the time when the needle was removed was recorded. Then 1 ml of blood was dispensed into each tube. The tubes were incubated at 37°C for 3 minutes. Each tube was gently inverted 360° at 30 second intervals. A full clot was defined as the condition when a tube could be inverted without blood flowing. If no clot was observed 20 minutes after the needle was removed from the sheep, the test was stopped.

Clotting time was defined as the amount of time between the removal of the needle from the sheep and the observation of a full clot. During each trial, the samples were tested in triplicate and the average of the three clotting times was defined as the Lee-White clotting time. If no clot was observed 20 minutes after the removal of the needle from the sheep, the average clotting time was calculated using the value of 20 minutes. In these cases, the actual clotting time will be underestimated.

Table I below shows the Lee-White clotting times for whole sheep blood with samples of setting bone substitute, samples of set bone substitute and corresponding controls.

**Table 1: Results are presented in minutes: seconds**

| **Blood Draw** | **1** | **2** | **3** | **4** | **5** | **6** | | **St.** |
|---|---|---|---|---|---|---|---|---|
| **Sheep Number** | **821** | **742** | **821** | **742** | **821** | **742** | **Mean** | **Dev.** |
| **Sample A (setting)** | 20+ | 20+ | 20+ | 20+ | 20+ | 20+ | 20:00+ | - |
| **Control: glass ball** | 08:15 | 07:29 | - | - | 06:36 | 08:06 | 07:36 | 00:45 |
| **Blank control: blood only** | - | - | 08:15 | 08:03 | 06:36 | 08:06 | 07:45 | 00:40 |

Twenty minutes after the needle was removed from the sheep, a full clot was not observed in any of the tubes containing Sample A.
Table 2 shows the Lee-White clotting time for contact with set Sample A bone substitute.

**Table 2**

| **Blood Draw** | **7** | **8** | **9** | **10** | **11** | **12** | | **St.** |
|---|---|---|---|---|---|---|---|---|
| **Sheep Number** | **821** | **742** | **821** | **742** | **821** | **742** | **Mean** | **Dev.** |
| **Sample A (set)** | 08:29 | 08:40 | 08:00 | 08:20 | 08:50 | 07:30 | 08:18 | 00:27 |
| **Control: glass ball** | 09:28 | 08:10 | - | - | 08:10 | 08:40 | 08:37 | 00:37 |
| **Blank control: blood only** | - | - | 09:20 | 08:50 | 08:30 | 08:50 | 08:52 | 00:18 |

The results indicate that contact with setting Sample A Radiopaque Calcium Phosphate Bone Substitute increases the Lee-White clotting time of whole sheep blood, but contact with set Sample A does not appear to affect it. Without being bound by a particular theory, it appears that while the cement is setting, the cement appears to be in a form that allows the leaching, release and/or diffusion of the ammonium salts to the blood. However, when the cement is set, the leaching, release and/or diffusion of the ammonium salts appears to be more limited.

### Study 2

The purpose of this study was to investigate the effects of several dilutions of diammonium hydrogen-phosphate solution (the liquid component of Sample A) on the Lee-White clotting time of whole sheep blood.

The procedure was the same as the one described for Study 1 except that 0.02ml of the Sample A liquid component, one of its dilutions, or 0.9% sodium chloride (control article) was placed in the blood collection tubes instead of the bone substitute samples.

Table 3 below shows the Lee-White clotting times for whole sheep blood with different dilutions of the Sample A liquid component and the control solution.

**Table 3: Results arc presented in minutes: seconds**

| **Date of Test** | **volume ratio (%)** | **reda cted** | **reda cted** | **reda cted** | **reda cted** | **reda cted** | **reda cted** | **reda cted** | **reda cted** | **reda cted** | **reda cted** | **Mean** |
|---|---|---|---|---|---|---|---|---|---|---|---|---|
| **Blood Draw Number** | | **5** | **6** | **7** | **8** | **9** | **10** | **9** | **1** | **2** | **3** | |
| **Sheep Number** | | **742** | **821** | **742** | **821** | **742** | **821** | **821** | **742** | **821** | **742** | |
| **Sample A liquid** | 2 | - | - | - | - | - | - | 20:00⁺ | 20:00⁺ | - | - | 20:00⁺ |
| **Sample A liquid** | 1 | - | - | - | - | - | - | - | - | 20:00⁺ | 20:00⁺ | 20:00⁺ |
| **Sample A liquid** | 0.75 | - | - | 20:00⁺ | 18:30* | - | - | - | - | - | - | 19:15 |
| **Sample A liquid** | 0.5 | - | - | 15:00 | 17:00* | 16:40* | 16: 10* | - | - | - | - | 16:12 |
| **Control: saline** | 2 | 07:30 | 08:30 | 09:00 | 08:10 | 08:40 | 07:00 | 07:40 | 08:27 | 09:20 | 07:52 | 08:13 |
| **Blank control: blood only** | - | 08:30 | 08:15 | - | - | 08:40 | 07:20 | 07:30 | 08:30 | 09:30 | 08:12 | 08:18 |

| | | | | | | | | | | | | |
|---|---|---|---|---|---|---|---|---|---|---|---|---|
| * During these tests, no clot was observed in one or two samples 20 minutes after the removal of the needle from the sheep. Thus the reported Lee-White clotting time is an underestimation of the actual clotting time. + During these tests, no clot was observed in all three samples 20 minutes after the removal of the needle from the sheep. | | | | | | | | | | | | |

The results indicate that the Lee-White clotting time for whole sheep blood is prolonged by contact with the Sample A liquid component. This effect appears to depend on the ratio of the Sample A liquid component volume to the volume of blood in the tube but in all cases, the presence of any Sample A liquid component appears to prolong coagulation time relative to the control.

### Study 3

The purpose of this study was to investigate the effects of several dilutions of diammonium hydrogen-phosphate solution (liquid component of Sample A) on the coagulation time of human plasma using the APTT test.

Clotting times of plasma samples were measured using the Amelung KC 4 A™ micro coagulation analyzer (Sigma Diagnostics, Germany). A cuvette was placed into a rotating well of the analyzer and a small stainless steel ball was dispensed into the cuvette. 100 µL of Pathromtin SL reagent were dispensed into the cuvette and then 100 µL of human plasma were added. For trials with the solution, 10 µL of water or of a solution dilution were added. After 2 minutes of incubation, 100 µL of CaCl₂ were dispensed into each cuvette. The analyzer timer was started immediately and clotting time was recorded when the timer stopped.

On each day, control plasma N (normal) and control plasma P (abnormal/ prolonged) were tested for quality control. At least two identical samples (duplicates) were tested simultaneously for each trial. The means and percent differences between duplicates were calculated. If the difference between duplicates was twenty percent or less, that trial's data was used for further analysis. Using this data, average values and standard deviations for all subjects were calculated.

Table 4 below shows the APPT values for normal human plasma from eight subjects with different dilutions of the ammonium solution. The chart below shows the averages and standard deviations of these times.

**Table 4**

| **NH₄ Conc. (mM)*** | **Subject APTT (sec)** | | | | | | | | | |
|---|---|---|---|---|---|---|---|---|---|---|
| | 1 | 2 | 3 | 4 | 5 | 6 | 7 | 8 | Average | St. Dev. |
| 40 | 236.5 | | | 154.4 | 186.2 | 111.9 | 166.2 | 255.8 | 185.2 | 53.5 |
| 30 | 79.8 | 52.8 | 64.2 | 64.6 | 86.9 | 58.5 | 57.5 | 76.4 | 67.6 | 12.1 |
| 20 | 64.5 | 46.3 | | 47.3 | 62.2 | 38.0 | 37.7 | 59.7 | 50.8 | 11.3 |
| 10 | 49.8 | 36.5 | 42.6 | 42.9 | 47.9 | 39.0 | 32.5 | 50.9 | 42.7 | 6.5 |
| 0** | | | | 38.3 | 42.7 | 34.1 | 29.6 | 35.2 | 36.0 | 4.9 |
| Plasma only | 42.9 | 33.8 | 33.7 | 35.4 | 43.4 | 34.0 | 30.4 | | 36.2 | 5.0 |

| | | | | | | | | | | |
|---|---|---|---|---|---|---|---|---|---|---|
| * The concentration of ammonium ions in the 310 µL total volume in each cuvette. ** 10 µL of water were added instead of the solution. | | | | | | | | | | |

The results indicate that the diammonium hydrogen-phosphate solution prolongs the clotting time of human plasma. This effect appears to depend on the concentration of ammonium ions with the higher the concentration of ammonium ions, the longer the time for coagulation to take place (see the Figure). The figure shows the average APTT value relative to the ammonium salt concentration.

Exemplary compositions of bone cements are given below. Composition 1 contains as the powder alpha-tricalcium phosphate, magnesium phosphate, magnesium hydrogen phosphate, strontium carbonate, barium sulfate in relative amounts 77.4, 14.3, 4.7, 3.6 and 0.0, respectively. The liquid composition for composition 1 is 3.5 M diammonium hydrogen phosphate in a relative amount of 0.5. Composition 2 contains as the powder alpha-tricalcium phosphate, magnesium phosphate, magnesium hydrogen phosphate, strontium carbonate, barium sulfate in relative amounts 77.4, 14.3, 4.7, 3.6 and 0.0, respectively. The liquid composition for composition 2 is 3.5 M diammonium hydrogen phosphate in a relative amount of 0.4. Composition 3 contains as the powder alpha-tricalcium phosphate, magnesium phosphate, magnesium hydrogen phosphate, strontium carbonate, barium sulfate in relative amounts 69.0, 12.7, 8.5, 3.2 and 6.5, respectively. The liquid composition is 3.5 M diammonium hydrogen phosphate in a relative amount of 0.4. All of these compositions work well to prevent blood clotting.

In an exemplary study, 10 grams of a powdered cement was mixed with a 3.5 molar aqueous (NH₄)₂HPO₄ solution at a liquid to powder ratio of 0.50 (ml liquid /g powder) to form a paste. Four sheep underwent implantations in L3, L4 and L5 with the resulting mixture. Implantation was made by a retroperitoneal lateral approach through the oblique abdominal muscle to the lateral aspect of the vertebral body (VB). All animal surgeries were performed under general anesthesia. The sheep that were treated with the cement did not undergo any deleterious clotting.

Accordingly, in an embodiment, the present invention discloses a method of inhibiting, delaying or preventing blood clotting comprising administering a pharmaceutical composition comprising an ammonium salt to an individual in need thereof. The ammonium salts can be one or more members selected from the group consisting of diammonium phosphate salts, ammonium phosphate salts, ammonium chloride salts, ammonium bromide salts, ammonium iodide salts, ammonium sulfate salts, dimethyl ammonium chloride salts, dimethyl ammonium methyl sulfate salts, dimethyl ammonium acetate salts, dipropyl ammonium phosphate salts, dimethyl ammonium nitrate salts, di(alkyl)dimethyl ammonium chloride salts, di(alkyl)dimethyl ammonium bromide salts, tetraalkyl ammonium salts, alkyl trimethyl ammonium salts, ammonium nitrate salts, ammonium thiocyanate salts, ammonium sulfaminate salts, dilauryl dimethyl ammonium chloride salts, distearyl dimethyl ammonium chloride salts, dimyristyl dimethyl ammonium chloride salts, dipalmityl dimethyl ammonium chloride salts, distearyl dimethyl ammonium chloride salts, stearamidopropyl PG-diammonium chloride phosphate salts, stearamidopropyl ethyldiammonium ethosuifate salts, stearamidopropyl dimethyl (myristyl acetate) ammonium chloride salts, stearamidopropyl dimethyl cetearyl ammonium tosylate salts, stearamidopropyl dimethyl ammonium chloride salts, stearamidopropyl dimethyl ammonium lactate salts, and mixtures thereof.

In a variation of the method, the composition further comprises one or members from the group consisting of warfarin (Coumadin), heparin, Anisindione, Sintrom (Acenocoumarol), Warfilone, Miradon, 1,3-indanediones, antithrombin, protein C, and thrombomodulin. If heparin is present, it may be present as an ammonium salt.

In another embodiment, the invention discloses an anticoagulants that comprises an ammonium salt wherein the ammonium salt is not an ammonium salt of citrate, an ammonium salt of heparin, an ammonium salt of tartrate, an ammonium salt of oxalate, or an ammonium salt of heparin. In a variation of this embodiment, the ammonium salt may be one or more members selected from the group consisting of diammonium phosphate salts, ammonium phosphate salts, ammonium chloride salts, ammonium bromide salts, ammonium iodide salts, ammonium sulfate salts, dimethyl ammonium chloride salts, dimethyl ammonium methyl sulfate salts, dimethyl ammonium acetate salts, dipropyl ammonium phosphate salts, dimethyl ammonium nitrate salts, di(alkyl)dimethyl ammonium chloride salts, di(alkyl)dimethyl ammonium bromide salts, tetraalkyl ammonium salts, alkyl trimethyl ammonium salts, ammonium nitrate salts, ammonium thiocyanate salts, ammonium sulfaminate salts, dilauryl dimethyl ammonium chloride salts, distearyl dimethyl ammonium chloride salts, dimyristyl dimethyl ammonium chloride salts, dipalmityl dimethyl ammonium chloride salts, distearyl dimethyl ammonium chloride salts, stearamidopropyl PG-diammonium chloride phosphate salts, stearamidopropyl ethyldiammonium ethosulfate salts, stearamidopropyl dimethyl (myristyl acetate) ammonium chloride salts, stearamidepropyl dimethyl cetearyl ammonium tosylate salts, stearamidopropyl dimethyl ammonium chloride salts, stearamidopropyl dimethyl ammonium lactate salts, and mixtures thereof.

In an alternate embodiment, the present invention discloses a pharmaceutical anticoagulant composition comprising an ammonium salt in connection with one or more of a pharmaceutically acceptable diluents, carriers, excipients, and/or other pharmaceutically acceptable salts. This embodiment may further comprise one or more other anticoagulants.
The one or more other anticoagulants may be selected amongst an ammonium salt of citrate, an ammonium salt of heparin, an ammonium salt of tartrate, an ammonium salt of oxalate, warfarin (Coumadin), heparin, Anisindione, Sintrom (Acenocoumarol), Warfilone, Miradon, 1,3-indanediones, antithrombin, protein C, and thrombomodulin.

In another embodiment, the present invention discloses a medical device that comprises a polymer and an ammonium sulfate salt as part of the medical device. In a variation of this embodiment, the medical device is a non-resorbable polymeric extruded medical device. In a further variation of this embodiment, the ammonium salt is concentrated primarily on the surface of the medical device. The ammonium salt may be selected from amongst diammonium phosphate salts, ammonium phosphate salts, ammonium chloride salts, ammonium bromide salts, ammonium iodide salts, ammonium sulfate salts, dimethyl ammonium chloride salts, dimethyl ammonium methyl sulfate salts, dimethyl ammonium acetate salts, dipropyl ammonium phosphate salts, dimethyl ammonium nitrate salts, di(alkyl)dimethyl ammonium chloride salts, di(alkyl)dimethyl ammonium bromide salts, tetraalkyl ammonium salts, alkyl trimethyl ammonium salts, ammonium nitrate salts, ammonium thiocyanate salts, ammonium sulfaminate salts, dilauryl dimethyl ammonium chloride salts, distearyl dimethyl ammonium chloride salts, dimyristyl dimethyl ammonium chloride salts, dipalmityl dimethyl ammonium chloride salts, distearyl dimethyl ammonium chloride salts, stearamidopropyl PG-diammonium chloride phosphate salts, stearamidopropyl ethyldiammonium ethosulfate salts, stearamidopropyl dimethyl (myristyl acetate) ammonium chloride salts, stearamidopropyl dimethyl cetearyl ammonium tosylate salts, stearamidopropyl dimethyl ammonium chloride salts, stearamidopropyl dimethyl ammonium lactate salts, and mixtures thereof. The medical device may also contain one or more of an ammonium salt of citrate, an ammonium salt of heparin, an ammonium salt of tartrate, an ammonium salt of oxalate, warfarin (Coumadin), heparin, Anisindione, Sintrom (Acenocoumarol), Warfilone, Miradon, 1,3-indanediones, antithrombin, protein C, and thrombomodulin. In a variation of this embodiment, the medical device is used in a surgical procedure.

In yet another embodiment, the invention discloses a non-bioresorbable polymeric medical device or implant comprising an ammonium salt wherein the non-bioresorbable polymeric medical device or implant is designed to allow the ammonium salt to leach into a patient's circulatory system. In a variation of this embodiment, the non-bioresorbable polymeric medical device or implant is extruded. Further, the non-bioresorbable polymeric implant may be a catheter. In another variation, the medical device or implant may be molded or thermoformed. When the medical device or implant is thermoformed it may be, for example a blood filter, a drip chamber, or a blood oxygenator, or other similar devices.

In another embodiment, the invention discloses a bioresorbable polymeric medical device, bioresorbable gel or hydrogel, or permeable membrane wherein said bioresorbable polymeric medical device, bioresorbable gel or hydrogel, or permeable membrane comprises an ammonium salt. In a variation of this embodiment, the bioresorbable polymeric medical device is a bioresorbable suture and the bioresorbable suture may be made of a polylactic acid/polyglycic acid copolymer.

In an alternate embodiment, the present invention discloses a bone cement that also is an effective anticoagulant, wherein said bone cement comprises one or more ammonium salts. The bone cement may also contain one or more other coagulants such as ammonium salt of citrate, an ammonium salt of heparin, an ammonium salt of tartrate, an ammonium salt of oxalate, warfarin (Coumadin), heparin, Anisindione, Sintrom (Acenocoumarol), Warfilone, Miradon, 1,3-indanediones, antithrombin, protein C, and thrombomodulin.
In a variation of this embodiment, the bone cement may further comprise one or more antibiotics wherein the one or more antibiotics include gentamicin, gentamicin sulfate, erythromycin, tobramycin, vancomycin, cefazolin, oxacillin, cefotaxime, colistin, clindamycin, and fusidic acid. Moreover, the bone cement may further comprise one or more anticancer agents. Examples of these anticancer agents include 6-mercaptopurine, methotrexate and cisplatin. Other medicines can also be added to the bone cement.

A plurality of embodiments have been disclosed above. It should be recognized that further embodiments of the present invention are contemplated such that any one or more feature from any embodiment that is disclosed can be added to any one or more features from any other disclosed embodiment. In other words, the invention is not to be limited by any of the embodiments of the above description.

## Claims

1. An ammonium salt for use in inhibiting, delaying or preventing blood clotting, the use comprising administering the ammonium salt to an individual to inhibit, delay or prevent blood clotting, wherein the ammonium salt is one or more members selected from the group consisting of diammonium phosphate salts, ammonium phosphate salts, ammonium chloride salts, ammonium bromide salts, ammonium iodide salts, ammonium sulfate salts, ammonium nitrate salts, ammonium thiocyanate salts, ammonium sulfaminate salts, and mixtures thereof.

2. An ammonium salt for use according to Claim 1, wherein the use is in a medical device and comprises administering the ammonium salt in the medical device to an individual to inhibit, delay or prevent blood clotting.

3. An ammonium salt for use according to Claim 2, wherein the use is in a bone cement.

4. An ammonium salt for use according to any one of Claims 1-3, further comprising one or members from the group consisting of warfarin (Coumadin), heparin, Anisindione, Sintrom (Acenocoumarol), Warfilone, Miradon, 1,3-indanediones, antithrombin, protein C, and thrombomodulin.

5. An ammonium salt for use according to Claim 4, further comprising heparin and the heparin is present as an ammonium salt.

6. An ammonium salt for use according to any one of Claims 1-3, wherein the ammonium salt is one or more members selected from the group consisting of diammonium phosphate salts, ammonium phosphate salts, ammonium chloride salts, ammonium bromide salts, ammonium iodide salts, and ammonium sulfate salts; wherein, optionally, the ammonium salt is a diammonium phosphate salt.

7. An ammonium salt for use according to Claim 3, wherein the ammonium salt is provided in the bone cement; wherein, optionally, the bone cement further comprises one or more antibiotics selected from the group consisting of gentamicin, gentamicin sulfate, erythromycin, tobramycin, vancomycin, cefazolin, oxacillin, cefotaxime, colistin, clindamycin, and fusidic acid; wherein, further optionally, the bone cement further comprises one or more anticancer agents and the one or more anticancer agents is selected from the group consisting of 6-mercaptopurine, methotrexate and cisplatin.

8. An ammonium salt for use according to Claim 1, wherein the ammonium salt is provided in connection with one or more of a pharmaceutically acceptable diluent, carrier, excipient, and/or other pharmaceutically acceptable salt.

9. An ammonium salt for use according to any one of Claims 1-3 or 8, further comprising one or more anticoagulants selected from the group consisting of an ammonium salt of citrate, an ammonium salt of heparin, an ammonium salt of tartrate, an ammonium salt of oxalate, warfarin (Coumadin), heparin, Anisindione, Sintrom (Acenocoumarol), Warfilone, Miradon, 1,3-indanediones, antithrombin, protein C, and thrombomodulin.

10. An ammonium salt for use according to Claim 2, wherein the medical device is selected from the group consisting of a non-bioresorbable polymeric medical device, a bioresorbable polymeric medical device, a bioresorbable gel, hydrogel and an implant, and wherein said medical device is designed to allow the ammonium salt to leach into a patient's circulatory system.

11. An ammonium salt for use according to Claim 3, wherein the bone cement comprises a liquid portion and a powder portion, and wherein the ammonium salt is present in one or other of the liquid portion or the powder portion.

12. An ammonium salt for use according to any one of Claims 1, 2, 3, or 6, wherein the ammonium salt is diammonium hydrogen phosphate.

## Patentansprüche

1. Ammoniumsalz zur Verwendung bei der Hemmung, Verzögerung oder Verhinderung der Blutgerinnung, wobei die Verwendung das Verabreichen des Ammoniumsalzes an eine Person, um die Blutgerinnung zu hemmen, zu verzögern oder zu verhindern, umfasst, wobei das Ammoniumsalz ein oder mehrere aus der Gruppe ausgewählte Mitglieder ist, die aus Diammoniumphosphatsalzen, Ammoniumphosphatsalzen, Ammoniumchloridsalzen, Ammoniumbromidsalzen, Ammoniumiodidsalzen, Ammoniumsulfatsalzen, Ammoniumnitratsalzen, Ammoniumthiocyanatsalzen, Ammoniumsulfaminatsalzen und Gemischen davon besteht.

2. Ammoniumsalz zur Verwendung nach Anspruch 1, wobei die Verwendung in einer medizinischen Vorrichtung ist und das Verabreichen des Ammoniumsalzes in der medizinischen Vorrichtung an eine Person, um die Blutgerinnung zu hemmen, zu verzögern oder zu verhindern, umfasst.

3. Ammoniumsalz zur Verwendung nach Anspruch 2, wobei die Verwendung in einem Knochenzement ist.

4. Ammoniumsalz zur Verwendung nach einem der Ansprüche 1 - 3, das weiterhin ein oder mehrere Mitglieder aus der Gruppe umfasst, die aus Warfarin (Coumadin), Heparin, Anisindion, Sintrom (Acenocumarol), Warfilone, Miradon, 1,3-Indandionen, Antithrombin, Protein C und Thrombomodulin besteht.

5. Ammoniumsalz zur Verwendung nach Anspruch 4, das weiterhin Heparin umfasst und wobei das Heparin als ein Ammoniumsalz vorliegt.

6. Ammoniumsalz zur Verwendung nach einem der Ansprüche 1 - 3, wobei das Ammoniumsalz ein oder mehrere aus der Gruppe ausgewählte Mitglieder ist, die aus Diammoniumphosphatsalzen, Ammoniumphosphatsalzen, Ammoniumchloridsalzen, Ammoniumbromidsalzen, Ammoniumiodidsalzen und Ammoniumsulfatsalzen besteht; wobei gegebenenfalls das Ammoniumsalz ein Diammoniumphosphatsalz ist.

7. Ammoniumsalz zur Verwendung nach Anspruch 3, wobei das Ammoniumsalz in dem Knochenzement bereitgestellt ist; wobei gegebenenfalls der Knochenzement weiterhin ein oder mehrere aus der Gruppe ausgewählte Antibiotika umfasst, die aus Gentamicin, Gentamicinsulfat, Erythromycin, Tobramycin, Vancomycin, Cefazolin, Oxacillin, Cefotaxim, Colistin, Clindamycin und Fusidinsäure besteht; wobei weiterhin gegebenenfalls der Knochenzement weiterhin eine oder mehrere antineoplastische Substanzen umfasst und die eine oder die mehreren antineoplastischen Substanzen aus der Gruppe ausgewählt ist bzw. sind, die aus 6-Mercaptopurin, Methotrexat und Cisplatin besteht.

8. Ammoniumsalz zur Verwendung nach Anspruch 1, wobei das Ammoniumsalz in Verbindung mit einem oder mehreren von einem pharmazeutisch unbedenklichen Verdünnungsmittel, einem pharmazeutisch unbedenklichen Trägerstoff, einem pharmazeutisch unbedenklichen Hilfsstoff und/oder einem anderen pharmazeutisch unbedenklichen Salz bereitgestellt wird.

9. Ammoniumsalz zur Verwendung nach einem der Ansprüche 1 - 3 oder 8, das weiterhin ein oder mehrere aus der Gruppe ausgewählte Antikoagulantien umfasst, die aus einem Ammoniumsalz von Citrat, einem Ammoniumsalz von Heparin, einem Ammoniumsalz von Tartrat, einem Ammoniumsalz von Oxalat, Warfarin (Coumadin), Heparin, Anisindion, Sintrom (Acenocumarol), Warfilone, Miradon, 1,3-Indandionen, Antithrombin, Protein C und Thrombomodulin besteht.

10. Ammoniumsalz zur Verwendung nach Anspruch 2, wobei die medizinische Vorrichtung aus der Gruppe ausgewählt ist, die aus einer nicht bioresorbierbaren polymeren medizinischen Vorrichtung, einer bioresorbierbaren polymeren medizinischen Vorrichtung, einem bioresorbierbaren Gel, Hydrogel und einem Implantat besteht, und wobei die medizinische Vorrichtung dazu entworfen ist zu ermöglichen, dass das Ammoniumsalz in das Kreislaufsystem eines Patienten einsickert.

11. Ammoniumsalz zur Verwendung nach Anspruch 3, wobei der Knochenzement einen flüssigen Anteil und einen Pulveranteil umfasst und wobei das Ammoniumsalz in dem einen oder dem anderen des flüssigen Anteils oder des Pulveranteils vorliegt.

12. Ammoniumsalz zur Verwendung nach einem der Ansprüche 1, 2, 3 oder 6, wobei das Ammoniumsalz Diammoniumhydrogenphosphat ist.

## Revendications

1. Sel d'ammonium pour une utilisation dans l'inhibition, le retard ou la prévention de la coagulation sanguine, l'utilisation comprenant l'administration du sel d'ammonium à un individu pour inhiber, retarder ou prévenir la coagulation sanguine, le sel d'ammonium étant constitué par un ou plusieurs éléments choisis dans le groupe consistant en les sels phosphates de diammonium, les sels phosphates d'ammonium, les sels chlorures d'ammonium, les sels bromures d'ammonium, les sels iodures d'ammonium, les sels sulfates d'ammonium, les sels nitrates d'ammonium, les sels thiocyanates d'ammonium, les sels sulfaminates d'ammonium et leurs mélanges.

2. Sel d'ammonium pour une utilisation selon la revendication 1, dans lequel l'utilisation est dans un dispositif médical et comprend l'administration du sel d'ammonium dans le dispositif médical à un individu pour inhiber, retarder ou prévenir la coagulation sanguine.

3. Sel d'ammonium pour une utilisation selon la revendication 2, dans lequel l'utilisation est dans un ciment osseux.

4. Sel d'ammonium pour une utilisation selon l'une quelconque des revendications 1-3, comprenant en outre un ou plusieurs éléments du groupe consistant en la warfarin (Coumadine), l'héparine, l'Anisindione, le Sintrom (Acénocoumarol), la Warfilone, le Miradon, les 1,3-indanediones, l'antithrombine, la protéine C et la thrombomoduline.

5. Sel d'ammonium pour une utilisation selon la revendication 4, comprenant en outre de l'héparine et l'héparine est présente en tant que sel d'ammonium.

6. Sel d'ammonium pour une utilisation selon l'une quelconque des revendications 1-3, dans lequel le sel d'ammonium est un ou plusieurs éléments choisis dans le groupe consistant en les sels phosphates de diammonium, les sels phosphates d'ammonium, les sels chlorures d'ammonium, les sels bromures d'ammonium, les sels iodures d'ammonium et les sels sulfates d'ammonium ; dans lequel, facultativement, le sel d'ammonium est un sel phosphate de diammonium.

7. Sel d'ammonium pour une utilisation selon la revendication 3, dans lequel le sel d'ammonium est disposé dans le ciment osseux ; dans lequel, facultativement, le ciment osseux comprend en outre un ou plusieurs antibiotiques choisis dans le groupe consistant en la gentamicine, le sulfate de gentamicine, l'érythromycine, la tobramycine, la vancomycine, la céfazoline, l'oxacilline, le céfotaxime, la colistine, la clindamycine et l'acide fusidique ; dans lequel, encore facultativement, le ciment osseux comprend en outre un ou plusieurs agents anticancer et le ou les agents anticancer sont choisis dans le groupe consistant en la 6-mercaptopurine, le méthotrexate et le cisplatine.

8. Sel d'ammonium pour une utilisation selon la revendication 1, dans lequel le sel d'ammonium est fourni en liaison avec un ou plusieurs parmi un diluant, support, excipient pharmaceutiquement acceptable, et/ou autre sel pharmaceutiquement acceptable.

9. Sel d'ammonium pour une utilisation selon l'une quelconque des revendications 1-3 ou 8, comprenant en outre un ou plusieurs anticoagulants choisis dans le groupe consistant en un sel d'ammonium de citrate, un sel d'ammonium d'héparine, un sel d'ammonium de tartrate, un sel d'ammonium d'oxalate, la warfarine (Coumadine), l'héparine, l'Anisindione, le Sintrom (Acénocoumarol), la Warfilone, le Miradon, les 1,3-indanediones, l'antithrombine, la protéine C et la thrombomoduline.

10. Sel d'ammonium pour une utilisation selon la revendication 2, dans lequel le dispositif médical est choisi dans le groupe consistant en un dispositif médical polymère non-biorésorbable, un dispositif médical polymère biorésorbable, un gel biorésorbable, de l'hydrogel et un implant, et dans lequel ledit dispositif médical est agencé pour permettre au sel d'ammonium de se lixivier dans le système circulatoire d'un patient.

11. Sel d'ammonium pour une utilisation selon la revendication 3, dans lequel le ciment osseux comprend une partie liquide et une partie pulvérulente, et dans lequel le sel d'ammonium est présent dans l'une ou l'autre de la partie liquide ou de la partie pulvérulente.

12. Sel d'ammonium pour une utilisation selon l'une quelconque des revendications 1, 2, 3 ou 6, dans lequel le sel d'ammonium est l'hydrogéno phosphate de diammonium.
